# EUROPÄISCHE PATENTSCHRIFT

(11) **EP 3 220 980 B1**
(45) Veröffentlichungstag und Bekanntmachung des Hinweises auf die Patenterteilung: **24.02.2021**
(21) Anmeldenummer: 15812966.8
(22) Anmeldetag: 19.11.2015
(51) Int. Cl.: A61M 5/31, A61J 1/06, A61M 5/28

(54) **MEDIKAMENTENEINRICHTUNG**
DRUG DELIVERY DEVICE
DISPOSITIF D'ADMINISTRATION DE MÉDICAMENT

(30) Priorität: 20.11.2014 DE 102014223693
(43) Veröffentlichungstag der Anmeldung: 27.09.2017
(73) Patentinhaber: Vetter Pharma-Fertigung GmbH & Co. KG, 88212 Ravensburg (DE)
(72) Erfinder: ROEDLE, Tilman, 88364 Wolfegg (DE)
(74) Vertreter: Kordel, Mattias
(86) Internationale Anmeldenummer: PCT/EP2015/077069
(87) Internationale Veröffentlichungsnummer: WO 2016/079228

(56) Entgegenhaltungen:
- EP-A1- 1 825 877
- US-A- 5 810 001
- US-A1- 2003 052 788
- US-A1- 2010 305 506
- US-A1- 2012 078 181

## Beschreibung

Die Erfindung betrifft eine Medikamenteneinrichtung mit einer Karpule oder Spritze gemäß Oberbegriff des Anspruchs 1. Medikamenteneinrichtungen mit einer Karpule oder Spritze, einem zugehörigen Gehäuse und einer RFID (Radio-Frequency-Identification)-Einrichtung sind bekannt. Sie zeichnen sich dadurch aus, dass durch diese Einrichtung Informationen über eine in ein Gehäuse eingebrachte Karpule oder Spritze und beispielsweise über deren Inhalt gewonnen werden können. Es hat sich gezeigt, dass in vielen Fällen die Signalübertragung zwischen dem RFID-Chip und der RFID-Ausleseeinheit der RFID-Einrichtung nicht zufriedenstellend ist und dadurch die gewünschten Informationen oft nicht zugänglich sind. EP 1 825 877 A1 und US 2010/305506 A1 offenbaren jeweils eine Medikamenteneinrichtung mit RFID-Kommunikation zwischen einem Medikamentenbehälter und einem zugehörigen Injektorgehäuse. US 2003/052788 A1 offenbart RFID-Tags mit Spiralantenne.

Aufgabe der Erfindung ist es daher, eine Medikamenteneinrichtung der oben genannten Art zu schaffen, bei der eine erhöhte Funktionssicherheit gewährleistet ist.

Zur Lösung dieser Aufgabe wird eine Medikamenteneinrichtung vorgeschlagen, welche die in Anspruch 1 genannten Merkmale aufweist. Die Medikamenteneinrichtung weist demnach eine Karpule oder Spritze mit einem zugehörigen Gehäuse auf, die eine Längsbeziehungsweise Mittelachse umfassen, außerdem eine RFID-Einrichtung mit einem Chip und einer Ausleseeinheit. Dabei ist der Chip der Karpule oder Spritze und die Ausleseeinheit dem Gehäuse oder umgekehrt zugeordnet. Sowohl der Chip als auch die Ausleseeinheit sind mit einer Antenne versehen.

Die Medikamenteneinrichtung zeichnet sich dadurch aus, dass die Antennen jeweils als Spule ausgebildet und derart ausgerichtet sind, dass sie koaxial zueinander und zur Längsachse der Karpule sowie zur Mittelachse des Gehäuses ausgerichtet sind. Durch diese Anordnung der Antennen der RFID-Einrichtung ist sichergestellt, dass eine optimale Signalübertragung von dem Chip zur Ausleseeinheit gewährleistet ist, sodass - insbesondere unabhängig von einer Relativ-Drehposition der Karpule zum Gehäuse - eine sichere Informationsübertragung möglich ist.

Es ist vorgesehen, dass die erste Antenne des RFID-Chips und/oder die zweite Antenne der RFID-Ausleseeinheit mittels eines Schrumpfschlauchs an der Karpule beziehungsweise dem Gehäuse anbringbar sind. Es ist damit auf einfache Weise möglich, die Antennen exakt an einer gewünschten Position anzuordnen und dann auf leichte Weise zu befestigen.

Bei einem weiteren bevorzugten Ausführungsbeispiel ist vorgesehen, dass die Spule der ersten und/oder zweiten Antenne als geschlossener Ring oder als Ringsegment oder als Spirale ausgebildet ist. Es ist dabei möglich, die beiden Antennen des RFID-Chips und der RFID-Ausleseeinheit verschieden auszubilden.

Weitere Ausgestaltungen ergeben sich aus den Unteransprüchen. Die Erfindung wird im Folgenden anhand der Zeichnung näher erläutert. Es zeigen:
- Figur 1: eine Prinzipskizze einer Medikamenteneinrichtung mit einer Karpule und einem Gehäuse, die beide mit einer Antenne versehen sind,
- Figur 2: eine Prinzipskizze einer mit einem Schrumpfschlauch kombinierten Antenne mit einem RFID-Chip,
- Figur 3: eine Prinzipskizze einer Explosionsdarstellung eines Schrumpfschlauchs gemäß Figur 2 mit einer Karpule und
- Figur 4: eine Prinzipskizze einer Medikamenteneinrichtung mit einer Spritze mit einer Antenne in Explosionsdarstellung.

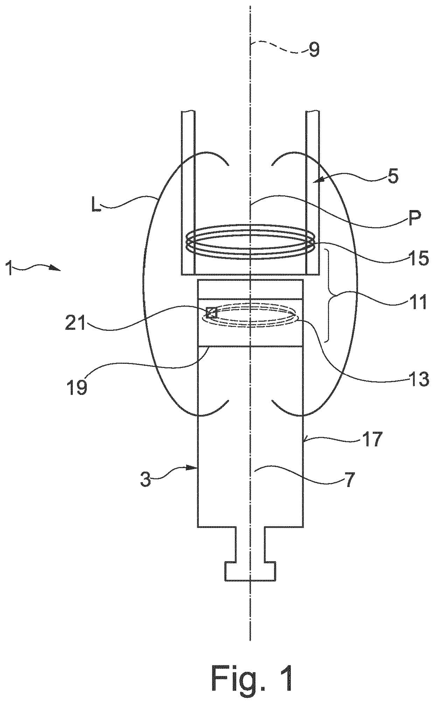

Figur 1 zeigt eine Prinzipskizze einer Medikamenteneinrichtung 1, welche eine Karpule 3 sowie ein Gehäuse 5, welches hier abgebrochen dargestellt ist, umfasst. Bei der Medikamenteneinrichtung 1 kann es sich beispielsweise um einen Pen handeln, der zur Verabreichung eines Medikaments dient, welches sich im Inneren der Karpule 3 befindet.

Gemäß Figur 1 weist die Karpule 3 eine Längsachse 7 auf, das Gehäuse 5 eine Mittelachse 9. Es ist ersichtlich, dass diese beiden Achsen koaxial zueinander angeordnet sind, also zusammenfallen.

Die Medikamenteneinrichtung 1 weist eine RFID-Einrichtung 11 mit einer ersten Antenne 13 sowie einer zweiten Antenne 15 auf. Diese sind beide als Spule ausgebildet. Beispielhaft ist vorgesehen, dass die erste Antenne 13 schraubenlinienförmig ausgebildet ist, während die zweite Antenne 15 ringförmig, insbesondere als Spirale, realisiert ist. Die Ausgestaltung der Antennen wird so gewählt, wie es für die Anbringung der Antennen vorteilhaft ist.

In Figur 1 ist die erste Antenne 13 schraubenlinienförmig ausgebildet, weil sie damit optimal auf der Außenfläche der Karpule 3 angebracht werden kann. Dabei ist es möglich, die erste Antenne 13 auf der Außenfläche 17 auf beliebige Weise zu befestigen beziehungsweise auszubilden. Denkbar ist es überdies, die erste Antenne in die Wand der Karpule 3 zu integrieren. Es ist also möglich, auf der Außenfläche 17 eine Antenne aufzudrucken, aufzukleben, oder aber, wie es hier erfindungsgemäß vorgesehen ist, mittels eines Schrumpfschlauchs 19 zu befestigen. Darauf wird unten noch näher eingegangen.

Die erste Antenne 13 ist mit einem RFID-Chip 21 gekoppelt, während die zweite Antenne 15 mit einer hier nicht dargestellten RFID-Ausleseeinheit gekoppelt ist.

Die zweite Antenne 15 ist hier beispielhaft als Spirale ausgebildet. Es ist sehr wohl möglich, auch diese zweite Antenne 15 schraubenlinienförmig auszulegen und außen auf der Wand des Gehäuses 5 oder auf dessen Innenseite vorzusehen, oder sie in die Wand des Gehäuses 5 zu integrieren.

Bei dem hier dargestellten Ausführungsbeispiel der Medikamenteneinrichtung 1 sind also zwei verschieden ausgelegte Antennen miteinander kombiniert, wobei die erste Antenne 13 der Karpule 3 und die zweite Antenne 15 dem Gehäuse 5 zugeordnet ist.

Aus Figur 1 wird deutlich, dass die erste Antenne 13 schraubenlinienförmig um eine gedachte Achse angeordnet ist, welche mit der Längsachse 7 der Karpule 3 zusammenfällt. Außerdem ist vorgesehen, dass die zweite Antenne 15 um eine gedachte Achse verläuft, welche mit der Mittelachse 9 des Gehäuses 5 zusammenfällt. Schließlich ist zu erkennen, dass die Achsen der beiden Antennen 13 und 15 sowie die Längsachse 7 der Karpule 3 und die Mittelachse 9 des Gehäuses 5 zusammenfallen, das heißt koaxial zueinander ausgerichtet sind.

Figur 1 ist zu entnehmen, dass die Felder der ersten und zweiten Antenne 13 und 15 koaxial zu der Längsachse 7 der Karpule 3 und der Mittelachse 9 des Gehäuses 5 verlaufen, wie dies durch einen koaxial zu der Längs- und Mittelachse verlaufenden Pfeil P verdeutlicht ist. Durch die äußeren Linien L ist angedeutet, wie das durch die Antennen 13 und 15 gebildete Feld außen verläuft.

Insbesondere ist ersichtlich, dass die Felder der Antennen 13 und 15 sich koaxial durchdringen, sodass eine Relativdrehung zwischen Karpule 3 und Gehäuse 5 keine Auswirkung auf die Übertragung von Energie an den RFID-Chip 21 hat und die Auslesung von in dem RFID-Chip 21 vorhandenen Daten durch die RFID-Ausleseeinheit optimal gewährleistet ist.

In Figur 1 sind die Antennen 13 und 15 als geschlossene Spulen dargestellt. Dabei ist es nicht erforderlich, dass die Antennen als geschlossener Ring ausgebildet sind. Denkbar ist es auch, ein Ringsegment als Antenne zu verwenden.

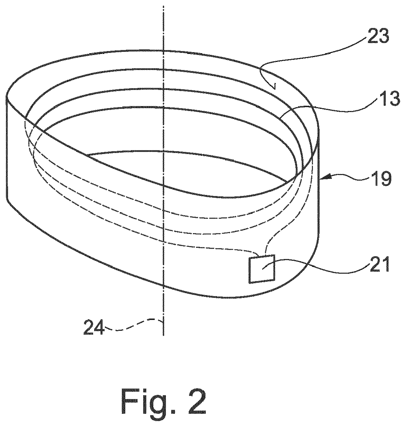

Figur 2 zeigt ein Teil der Medikamenteneinrichtung 1, nämlich in einer Prinzipskizze eine mit einem RFID-Chip gekoppelte Antenne mit einem Schrumpfschlauch. Gleiche und funktionsgleiche Elemente sind mit gleichen Bezugszeichen versehen, sodass insofern auf die vorangegangene Beschreibung verwiesen wird.

Figur 2 zeigt den aus Figur 1 ersichtlichen Schrumpfschlauch 19, der mit der ersten Antenne 13 versehen ist, welche mit einem RFID-Chip gekoppelt ist.

Es ist möglich, die Antenne 13 und den Schrumpfschlauch 19 getrennt auszubilden und die Antenne 13 zunächst auf die Außenfläche 17 beispielsweise einer Karpule 3 aufzulegen und anschließend mit dem Schrumpfschlauch 19 zu fixieren. Üblicherweise wird ein Schrumpfschlauch 19 mit einem Innendurchmesser gewählt, welcher größer ist als der Außendurchmesser der Karpule 3 beziehungsweise des Gegenstands, beispielsweise also auch des Gehäuses 5, an dem die Antenne 13 angebracht werden soll. Durch den größeren Innendurchmesser ist der Schrumpfschlauch 19 leicht anbringbar. Anschließend wird er beispielsweise erwärmt, wobei dessen Material so ausgelegt ist, dass es bei steigender Temperatur schrumpft, sodass sich der Innendurchmesser des Schrumpfschlauchs 19 reduziert und er fest auf der Außenfläche des Gegenstands anliegt, an dem die Antenne angebracht werden soll.

Denkbar ist es auch, den Schrumpfschlauch 19 aus Materialien herzustellen, die durch Einwirkung von Chemikalien, Licht, insbesondere UV-Licht oder dergleichen, schrumpfen.

Schrumpfschläuche sind grundsätzlich bekannt, sodass auf deren Ausgestaltung und Funktionsweise nicht weiter eingegangen wird.

Aus Figur 2 ist nach allem also beispielsweise ein Schrumpfschlauch 19 ersichtlich, auf dessen Innenseite 23 die erste Antenne 13 aufgebracht, aufgeklebt oder aufgelegt ist, sodass eine Einheit aus Antenne 13 und Schrumpfschlauch 19 besteht. Denkbar ist es darüber hinaus, die erste Antenne 13 in die Wandung des Schrumpfschlauchs 19 zu integrieren, vorzugsweise einschließlich des RFID-Chips 21.

Der Schrumpfschlauch 19, dadurch auch die erste Antenne 13, verlaufen um eine Achse 24.

Figur 2 ist zu entnehmen, dass die erste Antenne 13 schraubenlinienförmig ausgelegt ist. Sehr wohl denkbar ist es auch, eine spiralförmig ausgelegte Antenne mit dem Schrumpfschlauch 19 zu kombinieren, deren Windungen in einer Ebene liegen, auf der die Achse 24 senkrecht steht.

Die erste Antenne 13 und die aus Figur 1 ersichtliche zweite Antenne 15 sind hier als Schraubenlinie beziehungsweise als ringförmige Spule ausgebildet. Es sei hier ausdrücklich darauf hingewiesen, dass beide Antennen 13 und 15 auch zwei oder mehr Teilantennen aufweisen können, die vorzugsweise koaxial zueinander angeordnet sind.

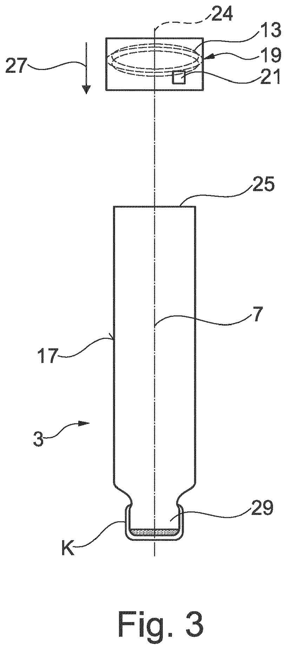

Figur 3 gibt in einer Prinzipskizze eine Explosionsdarstellung eines Schrumpfschlauchs gemäß Figur 2 mit einer Karpule wieder. Gleiche und funktionsgleiche Elemente sind mit gleichen Bezugszeichen versehen, sodass insofern auf die vorangegangene Beschreibung verwiesen wird.

Aus Figur 3 ist ersichtlich, dass der Schrumpfschlauch 19 mit der einen RFID-Chip 21 aufweisenden Antenne 13 im Ausgangszustand so groß ausgelegt ist, also einen derartigen Innendurchmesser aufweist, dass er problemlos über eine Karpule 3 geschoben werden kann. Er ist ohne weiteres über die Außenfläche 17 der Karpule 3 verlagerbar und in einer gewünschten Lage positionierbar. Vorzugsweise wird er in der Nähe des oberen Randes 25 der Karpule 3 angeordnet, wie dies aus Figur 1 ersichtlich ist.

Nach Aufsetzen des Schrumpfschlauchs 19 auf die Außenfläche 17 der Karpule 3 in Richtung des Pfeils 27, wird ein Schrumpfvorgang bezüglich des Schrumpfschlauchs 19 aktiviert, sodass dieser fest auf der Außenfläche 17 aufliegt und in einer gewünschten Position gehalten wird. Dabei wird auch der RFID-Chip 21 an der Karpule 3 fixiert, sodass Beschädigungen, insbesondere der Verbindung zwischen RFID-Chip und Antenne vermieden werden.

Der Schrumpfschlauch 19 ist koaxial zur Karpule 3 angeordnet, sodass also die Achse 24 des Schrumpfschlauchs 19 und die Längsachse 7 der Karpule 3 koaxial zueinander angeordnet sind und zusammenfallen.

Die Karpule 3 kann als übliche Einkammer-Karpule ausgelegt sein, oder aber auch als bekannte Doppelkammer-Karpule. Ihr Außendurchmesser ist so gewählt, dass sie ohne weiteres in das Innere des Gehäuses 7 einführbar ist. Sie kann an ihrem dem Rand 25 gegenüberliegenden Ende 29 mit einer Kanüle oder aber einem sonstigen Injektionssystem verbunden werden, um das in ihrem Inneren vorhandene Medikament einem Patienten verabreichen zu können.

Die Karpule 3 kann an ihrem unteren Ende auf beliebige bekannte Weise verschlossen werden, beispielsweise durch eine Kappe K, wie sie in Figur 3 angedeutet ist.

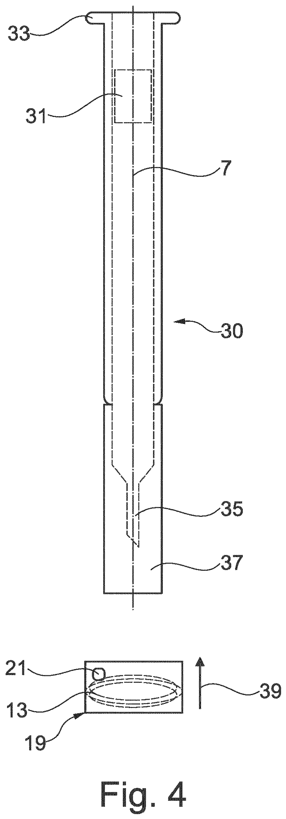

Figur 4 zeigt eine Prinzipskizze einer Medikamenteneinrichtung, die eine Spritze mit einer Antenne aufweist. Gleiche und funktionsgleiche Elemente sind mit gleichen Bezugszeichen versehen, sodass insofern auf die vorangegangene Beschreibung verwiesen wird.

Das in Figur 4 dargestellte Ausführungsbeispiel unterscheidet sich von dem in den Figuren 1 und 3 wiedergegebenen lediglich dadurch, dass anstelle einer Karpule hier eine Spritze 30 verwendet wird, die in Figur 4 beispielhaft als Einkammer-Spritze ausgelegt ist. Selbstverständlich ist es auch möglich, Zweikammer-Spritzen in Zusammenhang mit der hier beschriebenen Medikamenteneinrichtung zu verwenden.

Die hier dargestellte Spritze 30 wird mit einem Gehäuse 5 einer Medikamenteneinrichtung 1 kombiniert, wie sie anhand von Figur 1 erläutert wurde. Die Spritze 30 weist eine Längsachse 7 auf, die koaxial zu dem hier nicht dargestellten Gehäuse 5 einer Medikamenteneinrichtung 1 angeordnet ist.

Die Spritze 30 weist hier beispielhaft mindestens einen Stopfen 31 auf, der innerhalb der Spritze entlang deren Längsachse 7 verlagerbar ist. Sie umfasst außerdem an ihrem einen dem Gehäuse 5 der Medikamenteneinrichtung 1 zugewandten Ende einen umlaufenden Vorsprung 33 und an ihrem gegenüberliegenden Ende eine Kanüle 35 auf. Diese kann auch später auf die Spritze 30 aufgesetzt werden, bevor diese verwendet wird. Bei dem hier dargestellten Ausführungsbeispiel ist an dem dem Vorsprung 33 gegenüberliegenden Ende eine die Kanüle 35 übergreifende Schutzkappe 37 vorgesehen.

Spritzen 30 der hier angesprochenen Art sind bekannt und können verschieden ausgelegt sein. Auf die konkrete Ausgestaltung der Spritze 30 kommt es in Zusammenhang mit der hier angesprochenen Medikamenteneinrichtung nicht an.

Entscheidend ist, dass die Spritze 30 eine erste Antenne 13 aufweist, wie dies auch bei der Karpule 3 des ersten Ausführungsbeispiels der Medikamenteneinrichtung 1 der Fall ist. Hier ist die an der Spritze 30 vorgesehene erste Antenne 13 auch schraubenlinienförmig ausgebildet und mit einem RFID-Chip 21 verbunden.

Durch einen Pfeil 39 ist hier angedeutet, dass die erste Antenne 13 beziehungsweise ein Schrumpfschlauch 19 von unten, also von dem dem Vorsprung 33 gegenüberliegenden Ende der Spritze 30 auf deren Grundkörper aufgeschoben werden kann. Anschließend wird, wie anhand des ersten Ausführungsbeispiels beschrieben, der Schrumpfschlauch mit Wärme, Licht oder dergleichen beaufschlagt, sodass er auf die Spritze 30 aufgeschrumpft wird.

Grundsätzlich ist es sehr wohl möglich, die erste Antenne 13 unmittelbar auf den Grundkörper der Spritze 30 aufzubringen, sei es durch ein Druckverfahren, durch Aufkleben oder dergleichen. Schließlich ist es darüber hinaus möglich, die erste Antenne 13 in die Wand der Spritze 30 zu integrieren, die den mindestens einen Stopfen 31 aufnimmt. Hier gilt das für die Karpule 3 Gesagte entsprechend.

Am einfachsten ist es jedoch, die erste Antenne 13 mittels eines Schrumpfschlauchs 19 über den Grundkörper der Spritze 30 zu schieben und sie dort dann zu befestigen.

Zur Ausgestaltung der ersten Antenne 13, des Schrumpfschlauchs 19, der Integration der ersten Antenne 13 in den Schrumpfschlauch 19 und zu dessen Befestigung an der Spritze 30 wird im Übrigen auf die Erläuterungen verwiesen, die diesen Gesichtspunkt bei dem anhand der Figuren 1 bis 3 dargestellten Ausführungsbeispiel mit der Karpule 3 betreffen.

In Zusammenhang mit der Medikamenteneinrichtung 1 ist eine übliche RFID-Einrichtung vorgesehen, die, wie oben in Zusammenhang mit Figur 1 gesagt, eine bekannte Ausleseeinheit aufweist, die mit der zweiten Antenne 15 zusammenwirkt.

Üblicherweise umfasst die RFID-Ausleseeinheit eine Energiequelle, um über die zweite Antenne 15 und die erste Antenne 13 an der Karpule 3 oder Spritze 30 Energie dem RFID-Chip 21 zuzuführen und nach Aktivierung des Chips Daten auszulesen.

Dadurch, dass die beiden Antennen 13 und 15 koaxial zueinander angeordnet sind und auch koaxial zum Gehäuse 5 beziehungsweise der Karpule 3 oder Spritze 30, ist sichergestellt, dass die Energie von der RFID-Ausleseeinheit optimal an den RFID-Chip 21 übertragen wird. Gleichzeitig ist gewährleistet, dass Daten von dem RFID-Chip 21 sehr gut über die beiden Antennen 13 und 15 an die RFID-Ausleseeinheit übertragen werden.

Insbesondere wird auf diese Weise sichergestellt, dass der RFID-Chip 21 Informationen über die Karpule 3 oder Spritze 30, beispielsweise über deren Größe oder dergleichen, über das in der Karpule 3 oder Spritze 30 vorhandene Medikament und/oder über die Verabreichungsart des Medikaments überträgt. Auch kann beispielsweise das Restvolumen eines Medikaments in der Karpule 3 oder Spritze 30 an die RFID-Ausleseeinheit übermittelt werden.

Informationen über das Medikament an sich und die übliche oder für einen Patienten abgestimmte Dosierung können ebenfalls leicht übertragen werden.

Nach allem wird Folgendes deutlich:
Entscheidend für die hier beschriebene Ausgestaltung der Medikamenteneinrichtung 1 ist, dass zwei als Spule ausgebildete Antennen 13 und 15 einer RFID-Einrichtung 11 so ausgerichtet sind, dass sie koaxial zueinander und zur Längsachse 7 sowie zur Mittelachse 9 der Medikamenteneinrichtung 1 angeordnet sind.

Dabei kann eine der Antennen spiralförmig, auch als Ringsegment ausgebildet sein, während die andere schraubenlinienförmig ausgebildet ist. Auch ist eine spiralförmige oder schraubenlinienförmige Ausgestaltung beider Antennen 13 und 15 möglich.

Entscheidend ist die koaxiale Anordnung der Antennen, sodass Energie von einer zur anderen und Daten, vorzugsweise in der Gegenrichtung, optimal übertragbar sind.

Die Antennen können von außen auf die Wand einer Karpule 3 oder Spritze 30 beziehungsweise eines Gehäuses 5 aufgebracht werden und sind vorzugsweise mittels eines Schrumpfschlauchs 19 befestigbar. Denkbar ist es aber auch, eine oder beide Antennen auf der Innenseite der Karpule 3 beziehungsweise Spritze 30 oder des Gehäuses 5 vorzusehen, oder aber in die Wandung der Karpule 3 oder Spritze 30 und/oder des Gehäuses 5 zu integrieren.

Vorzugsweise ist die erste Antenne 13 auf der Außenfläche 17 der Karpule 3 oder Spritze 30 angeordnet, weil diese Montageart leicht durchführbar ist und der Innenraum der Karpule oder Spritze frei bleibt. Insbesondere die Innenwandung der Karpule oder Spritze sollte möglichst deshalb frei bleiben, weil in den Innenraum derselben mindestens ein Stopfen beziehungsweise Kolben eingeführt wird und dieser leicht und ungehindert verschiebbar sein soll.

## Patentansprüche

1. Medikamenteneinrichtung (1) mit
- einer eine Längsachse (7) aufweisenden sowie ein Medikament enthaltenden Karpule (3) oder Spritze (30),
- einem die Karpule (3) oder Spritze (30) aufnehmenden und eine Mittelachse (9) aufweisenden Gehäuse (5) und mit
- einer RFID-Einrichtung (11) umfassend
• mindestens einen RFID-Chip (21) mit einer ersten Antenne (13),
• mindestens einer RFID-Ausleseeinheit mit einer zweiten Antenne (15), wobei
• der RFID-Chip (21) der Karpule (3) beziehungsweise Spritze (30) und die RFID-Ausleseeinheit dem Gehäuse (5) zugeordnet ist, oder wobei der RFID-Chip (21) dem Gehäuse (5) und die RFID-Ausleseeinheit der Karpule (3) beziehungsweise Spritze (30) zugeordnet ist,
**dadurch gekennzeichnet, dass**
- die erste Antenne (13) des RFID-Chips (21) sowie die zweite Antenne (15) der RFID-Ausleseeinheit jeweils als Spule ausgebildet und derart ausgerichtet sind, dass sie koaxial zueinander und zur Längsachse (7) sowie zur Mittelachse (9) ausgerichtet sind, wobei
- die erste Antenne (13) und/oder die zweite Antenne (15) mittels eines Schrumpfschlauchs (19) an einer Umfangsfläche der Karpule (3) oder Spritze (30) beziehungsweise dem Gehäuse (5) angebracht sind.

2. Medikamenteneinrichtung nach Anspruch 1, **dadurch gekennzeichnet, dass** das Gehäuse (5) Teil eines Pens ist, mittels dessen das in der Karpule (3) oder Spritze (30) vorhandene Medikament verabreichbar ist.

3. Medikamenteneinrichtung nach einem der vorhergehenden Ansprüche, **dadurch gekennzeichnet, dass** der RFID-Chip (21) Informationen über das in der Karpule (3) oder Spritze (30) vorhandene Medikament und/oder über die Verabreichungsart des Medikaments umfasst.

4. Medikamenteneinrichtung nach Anspruch 1, **dadurch gekennzeichnet, dass** die erste Antenne (13) und/oder die zweite Antenne (15) mindestens zwei Teilantennen umfasst.

5. Medikamenteneinrichtung nach Anspruch 1, **dadurch gekennzeichnet, dass** die Spule der ersten und/oder zweiten Antenne (13,15) als geschlossener Ring, als Ringsegment oder als Spirale ausgebildet ist.

6. Medikamenteneinrichtung nach einem der vorhergehenden Ansprüche, **dadurch gekennzeichnet, dass** die Karpule (3) oder Spritze (30) als Ein- oder Zweikammerkarpule ausgelegt ist.

7. Medikamenteneinrichtung nach einem der vorhergehenden Ansprüche, **dadurch gekennzeichnet, dass** diese als Pen ausgebildet ist.

## Claims

1. A medication device (1), with
- a cartridge (3) or syringe (30) having a longitudinal axis (7) and containing a medicament,
- a housing (5) receiving the cartridge (3) or syringe (30) and having a center axis (9), and with
- an RFID device (11), comprising
• at least one RFID chip (21) with a first antenna (13),
• at least one RFID readout unit with a second antenna (15), wherein
• the at least one RFID chip (21) is assigned to the cartridge (3) or the syringe (30) and the at least one RFID readout unit is assigned to the housing (5), or wherein the at least one RFID chip (21) is assigned to the housing (5) and the at least one RFID readout unit is assigned to the cartridge (3) or the syringe (30),
**characterized in that**
- the first antenna (13) of the at least one RFID chip (21) and the second antenna (15) of the at least one RFID readout unit are each configured as a coil and aligned such that the first antenna (13) and the second antenna (15) are coaxial with one another and with the longitudinal axis (7) as well as with the center axis (9), wherein
- the first antenna (13) and/or the second antenna (15) are each mounted on a peripheral surface of the cartridge (3) or the syringe (30) or the housing (5) by means of a shrink hose (19).

2. A medication device (1) according to claim 1, **characterized in that** the housing (5) is part of a pen by means of which the medicament contained in the cartridge (3) or the syringe (30) can be administered.

3. The medication device according to any one of the preceding claims, **characterized in that** the at least one RFID chip (21) contains information about the medicament contained in the cartridge (3) or the syringe (30) and/or about the method of administration of the medicament.

4. The medication device according to claim 1, **characterized in that** the first antenna (13) and/or the second antenna (15) comprises at least two sub-antennas.

5. The medication device according to claim 1, **characterized in that** the coil of the first antenna (13) and/or the coil of the second antenna (15) is configured as a closed ring or as a ring segment or as a spiral.

6. The medication device according to any one of the preceding claims, **characterized in that** the cartridge (3) or the syringe (30) is designed as a single- or dual-chamber cartridge or syringe.

7. The medication device as set forth in any one of the preceding claims, **characterized in that** the medication device is configured as a pen.

## Revendications

1. Dispositif d'administration de médicament (1) présentant
- une cartouche (3) ou seringue (30) présentant un axe longitudinal (7) et contenant un médicament,
- un boîtier (5) logeant la cartouche (3) ou seringue (30) et présentant un axe central (9) et
- un dispositif RFID (11) comprenant
• au moins une puce RFID (21) présentant une première antenne (13)
• au moins une unité de lecture RFID présentant une deuxième antenne (15),
• la puce RFID (21) étant associée à la cartouche (3) et/ou seringue (30) et l'unité de lecture RFID au boîtier (5), ou la puce RFID (21) étant associée au boîtier (5) et l'unité de lecture RFID à la cartouche (3) et/ou seringue (30),
**caractérisé en ce que**
- la première antenne (13) de la puce RFID (21) et la deuxième antenne (15) de l'unité de lecture RFID sont respectivement conçues sous forme de bobines et orientées de telle manière à être alignées coaxialement l'une par rapport à l'autre et par rapport à l'axe longitudinal (7) ainsi que par rapport à l'axe central (9),
- la première antenne (13) et/ou la deuxième antenne (15) étant posées, au moyen d'une gaine rétractable (19), sur une surface périphérique de la cartouche (3) ou seringue (30) et/ou sur le boîtier (5).

2. Dispositif d'administration de médicament conformément à la revendication 1, **caractérisé en ce que** le boîtier (5) fait partie intégrante d'un stylo, au moyen duquel le médicament présent dans la cartouche (3) ou seringue (30) est administrable.

3. Dispositif d'administration de médicament conformément à l'une des revendications précédentes, **caractérisé en ce que** la puce RFID (21) contient des informations sur le médicament présent dans la cartouche (3) ou seringue (30) et/ou sur le mode d'administration du médicament.

4. Dispositif d'administration de médicament conformément à la revendication 1, **caractérisé en ce que** la première antenne (13) et/ou la deuxième antenne (15) comprend au moins deux antennes partielles.

5. Dispositif d'administration de médicament conformément à la revendication 1, **caractérisé en ce que** la bobine de la première et/ou deuxième antenne (13, 15) est conçue sous forme d'anneau fermé, de segment annulaire ou de spirale.

6. Dispositif d'administration de médicament conformément à l'une des revendications précédentes, **caractérisé en ce que** la cartouche (3) ou seringue (30) est conçue comme cartouche à un ou deux compartiments.

7. Dispositif d'administration de médicament conformément à l'une des revendications précédentes, **caractérisé en ce que** ce dernier est conçu sous forme de stylo.
